# EUROPÄISCHE PATENTSCHRIFT

(11) **EP 2 058 005 B1**
(45) Veröffentlichungstag und Bekanntmachung des Hinweises auf die Patenterteilung: **15.12.2010**
(21) Anmeldenummer: 08016498.1
(22) Anmeldetag: 18.09.2008
(51) Int. Cl.: A61K 39/35, A61K 38/04, C07K 14/415, C07K 7/08

(54) **Impfstoff zur Behandlung von Zöliakie und Verfahren zur Herstellung des Impfstoffes, Verwendung einer mit einem bakteriellen Toxin konjugierten Peptidsequenz zur Aktivierung des Immunsystems gegen Prolamine sowie Designerpeptid**
Inoculant for treating coeliac disease and method for producing the inoculant, application of a peptide sequence joined with a bacterial toxin for activating the immune system against prolamine and designer peptide
Vaccin destiné au traitement de l'intolérance au gluten et procédé de fabrication du vaccin, utilisation d'une séquence de peptides conjuguée à une toxine bactérienne pour activer le système immunitaire contre la prolamine et peptide de design

(30) Priorität: 18.09.2007 DE 102007044673
(43) Veröffentlichungstag der Anmeldung: 13.05.2009
(73) Patentinhaber: Zimmer, Klaus-Peter, Prof.Dr.med., 35390 Giessen (DE)
(72) Erfinder: Zimmer, Klaus-Peter, Prof.Dr.med., 35390 Giessen (DE)
(74) Vertreter: Walcher, Armin

(56) Entgegenhaltungen:
- WO-A1-99/54452
- WO-A2-03/104273
- WO-A2-2005/105129
- MAMONE ET AL: "Identification of a peptide from alpha-gliadin resistant to digestive enzymes: Implications for celiac disease" JOURNAL OF CHROMATOGRAPHY B: BIOMEDICAL SCIENCES & APPLICATIONS, ELSEVIER, AMSTERDAM, NL, Bd. 855, Nr. 2, 8. August 2007 (2007-08-08), Seiten 236-241, XP022191243 ISSN: 1570-0232
- SENGER STEFANIA ET AL: "Identification of immunodominant epitopes of alpha-gliadin in HLA-DQ8 transgenic mice following oral immunization." JOURNAL OF IMMUNOLOGY (BALTIMORE, MD. : 1950) 15 DEC 2005, Bd. 175, Nr. 12, 15. Dezember 2005 (2005-12-15), Seiten 8087-8095, XP009114320 ISSN: 0022-1767
- MAIURI L ET AL: "Association between innate response to gliadin and activation of pathogenic T cells in coeliac disease" LANCET THE, LANCET LIMITED. LONDON, GB, Bd. 362, Nr. 9377, 5. Juli 2003 (2003-07-05), Seiten 30-37, XP004436476 ISSN: 0140-6736
- HOLMGREN J ET AL: "MUCOSAL ADJUVANTS AND ANTI-INFECTION AND ANTI-IMMUNOPATHOLOGY VACCINES BASED ON CHOLERA TOXIN, CHOLERA TOXIN B SUBUNIT AND CPG DNA" EXPERT REVIEW OF VACCINES, FUTURE DRUGS, LONDON, GB, Bd. 2, Nr. 2, 1. April 2003 (2003-04-01), Seiten 205-217, XP009060650 ISSN: 1476-0584

## Beschreibung

Die Erfindung betrifft einen Impfstoff gemäβ Anspruch 1 und ein verfahren zur Herstellung dieses Impfstoffes zur Behandlung von Zöliakie gemäβ Anspruch 4 und die Verwendung gemäβ Anspruch 6. Darüber hinaus betrifft die vorliegende Erfindung ein Designerpeptid, bestehend aus der Aminosäuresequenz Pro Gly Glu Glu Glu Pro Phe Pro Pro Glu Glu Pro Tyr Pro:Glu Pro Glu Pro Phe Pro des α-Gliadins, die mit dem B-Pentamer des Choleratoxins konjugiert ist.

Zöliakie ist eine chronische Autoimmunerkrankung der Dünndarmschleimhaut, die bei genetisch anfälligen Individuen (HLA-DQ2/DQ8) durch eine immunologisch vermittelte Selbstzerstörung von Enterozyten bei Überempfindlichkeit gegen das Protein Gluten auftritt, welches auch als Klebereiweiß bekannt. Die Erkrankung, dass heißt die Hypersensitivität gegen Gluten bleibt lebenslang bestehen und ist zum Teil genetisch determiniert und kann derzeit nicht behandelt werden. Lediglich eine lebenslange glutenfreie Diät kann derzeit als die einzige gesicherte Möglichkeit zur Behandlung von Zöliakie empfohlen werden, wodurch der Darm wieder heilt und auch die Risiken der Langzeitfolgen sinken, wie beispielsweise im schlimmsten Fall das Auftreten eines Non-Hodgkin-Lymphoms oder von Darmkrebs. Aber auch die in Folge der Entzündung und ausgedehnten Zerstörung der Darmepithelzellen auftretenden Symptome, wie Gewichtsverlust, Durchfall, Erbrechen, Appetitlosigkeit und Vitaminmangel führen zum einen zu einer Entwicklungsstörung im Kindesalter und haben zum anderen zu Folge, dass die Lebensqualität der glutensensitiven Patienten extrem vermindert ist. Es kann also derzeit, wie schon oben beschrieben, nur eine glutenfreie Diät dazu führen, dass die betroffenen Patienten symptomfrei bleiben. Problematisch dabei ist, dass Gluten in vielen Getreidesorten, wie Weizen, Gerste, Roggen und Hafer, wie auch in deren botanisch verwandten Ursorten Dinkel, Grünkern, Kamut, Einkorn und Emmer vorkommt, wobei insbesondere die gängigen Getreidesorten vermehrt in der Lebensmittelindustrie eingesetzt werden. Es ist also extrem darauf zu achten, wie entsprechende Lebensmittel zusammengesetzt sind, da bereits kleine Mengen von Gluten den immunologischen Prozess der Selbstzerstörung der Enterozyten bei Gluten sensitiven Individuen auslöst. Da die Prävalenz, das heißt die Häufigkeit der symptomatischen Fälle im weltweiten Durchschnitt bei ungefähr 1:270 Individuen liegt, hat sich die Lebensmittelindustrie inzwischen auf die Gruppe der Patienten, die an Zöliakie leiden, eingestellt, so dass es inzwischen glutenfreie und auch als solche markierte Lebensmittelprodukte auf dem Markt gibt. Allerdings sind diese Produkte nur als begrenztes Sortiment verfügbar und können nur dazu beitragen, dass die glutenhypersensitiven Patienten symptomfrei leben, aber eine Heilung kann derzeit durch diese Maßnahmen nicht erreicht werden.

Ursächlich für die Hypersensitivität gegen das Gluten, sind toxische Peptidsequenzen, also Abschnitte des Glutens, die der alkohollöslichen Fraktion (Prolamine) angehören und als Gliadin bezeichnet werden, das aus einer Aminosäuresequenz besteht, die eine große Anzahl von Prolin und Glutamin aufweist. Insbesondere der hohe Prolingehalt macht diese Gluten- oder Gliadinpeptide resistent gegenüber dem üblichen Abbau bei der Verdauung, da diese Peptide durch ihre Prolinreste durch die üblichen Verdauungsenzyme nur unzureichend abbaubar sind. Dies führt bei entsprechend veranlagten Individuen mit einer Hypersensitivität gegen Gluten zu der oben bezeichneten Entzündung der Darmschleimhaut, die auf eine komplexe pathogene Reaktion des Immunsystems zurückzuführen ist. Denn durch die unvollständige Hydrolyse der prolin- und gluatminreichen Proteine werden antigene Epitope gebildet, die durch nichtspezifische Transcytose vom Intestinallumen in die darunter liegende Schleimhaut des Darmepithels transportiert werden. Bestimmte toxische Abschnitte, beispielsweise die Aminosäuresequenzen 31-49 und 57-72 des α-Gliadins, binden an in der Zellmembran der Enterozyten verankerte Glykoproteine, die zu den Immunglobulinen gehören und als Haupthistokompatibilitätskomplexe (HLA) bezeichnet werden. Diese Bindung wird noch dadurch verstärkt, dass aus der in der Peptidsequenz vorhandenen Aminosäure Glutamin durch Vermittlung des Enzyms Gewebstransglutaminase Glutaminsäure gebildet wird, wodurch das toxische Gliadin- Glutenpeptid in die von dem Haupthistokompatibilitätskomplex ausgebildete Tasche passt. Dies löst eine vermehrte Produktion von entzündungsauslösenden Botenstoffen, wie Interferon-γ, TNF-α, Interleukin-6 und Interleukin-2 aus, die in der Apoptose der Enterozyten, also im Absterben des Gewebes enden, beispielsweise eine Zottenatrophie zur Folge hat. Weshalb es zu dieser vermehrten Produktion von entzündungsauslösenden Botenstoffen kommt, ist derzeit noch nicht verstanden. Denn normaler Weise führen Antigene aus Bakterien oder aus dem Lebensmittel zu einer kontrollierten physiologischen Entzündung, die letztendlich in einer Toleranz gegenüber dem Antigen endet. Die bei der Zöliakie auftretenden Entzündungsprozesse laufen dagegen unkontrolliert ab, was, wie schon oben erwähnt, letztlich nicht verstanden wird.

Die Untersuchungen zur Behandlung von Zöliakie konzentrieren sich zur Zeit auf die Haupt-Antigenpräsentierende Funktion der DQ2/DQ8 positiven dendritischen Zellen der Lamina propria, während die in den Enterozyten ablaufenden Entzündungsauslösenden Prozesse nicht weiter Beachtung zur Entwicklung von Therapeutika gegen Zöliakie finden, geschweige denn über die Entwicklung eines Impfstoffes gegen Zöliakie nachgedacht wird.

Die WO 99/54452 A1 offenbart Verfahren und Substanzen zur Vorbeugung und Behandlung einer Autoimmunerkrankung, wobei ein Fusionsprotein aus der B-Untereinheit des nicht-toxischen Choleratoxins und einem Autoantigen bereitgestellt wird.

Senger S. et al. berichten über die Identifizierung eines immundominanten Epitops von α-Gliadin in HLA-DQ8 transgenen Mäusen nach oraler Immunisierung (J. Immunol. 2005, Seiten 8087 bis 8095).

Aufgabe der vorliegenden Erfindung ist es daher, einen Impfstoff gegen Zöliakie und ein Verfahren zur Herstellung des besagten Impfstoffes bereitzustellen, der es bei Gabe an genetisch veranlagte DQ2/DQ8 positive Individuen oder an glutensensitive Individuen ermöglicht, die bei Glutenaufnahme entstehenden Entzündungen der Schleimhaut zu unterdrücken oder die toxischen Peptidsequenzen (Antigene) immunologisch abzufangen, dass heißt, das Immunsystem der Zöliakie-Patienten auf die Antigene (toxische Peptidsequenzen) zu triggern.

Eine weitere Aufgabe der vorliegenden Erfindung ist die Verwendung eines Antigens, welches nach Gabe an Patienten, die an Zöliakie erkrankt sind oder die Veranlagung dazu besitzen, eine kontrollierte Immunantwort hervorruft, so dass die Patienten eine Toleranz gegen Prolamine, insbesondere gegen Gluten bzw. Gliadin entwickeln.

Schließlich ist es Aufgabe der vorliegenden Erfindung, einen Stoff zur Verfügung zu stellen, der zur Behandlung von Zöliakie einsetzbar ist.

Erfindungsgemäβ wird die voranstehende Aufgäbe durch einen Impfstoff zur Anwendung in der Behandlung von Zöliakie mit den Merkmalen des Anspruchs 1 gelöst.

Des Weiteren ist die obige Aufgabe im Hinblick auf die Herstellung eines Impfstoffes gegen Zöliakie durch ein Verfahren mit den Merkmalen des Anspruchs 4 gelöst.

Die weitere Aufgabe, nämlich die Verwendung eines Antigens, welches nach Gabe an Patienten, die an Zöliakie erkrankt sind oder erblich dazu veranlagt sind, eine kontrollierte Immunantwort hervorruft, wird durch die Verwendung gemäß Patentanspruch 6 gelöst.

Schließlich ist die Aufgabe, nämlich einen Stoff zur Verfügung zu stellen, der zur Behandlung von Zöliakie einsetzbar ist, durch ein Designerpeptid mit den Merkmalen des Anspruchs 7 gelöst.

In erfindungsgemäßer Weise ist erkannt worden, dass entgegen der derzeitigen Herangehensweise zur Entwicklung einer Therapie gegen Zöliakie, die sich auf die Mechanismen der Immunantwort der dendritischen Zellen des Darmepithels auf die toxischen Gluten- bzw. Gliadin-Peptidsequenzen beschränkt, ein gänzlich anderer Ansatz zum Erfolg führt, welcher die Präsentation der toxischen Peptidsequenzen als Antigene an den MHC-Klasse II Komplexen durch Vermittlung eines Adjuvans auf der Oberfläche der nährstoff-/antigenaufnehmenden Enterozyten vorsieht, wodurch eine Immunantwort, nämlich die Bildung von Antikörpern oder supprimierenden T-Lymphozyten ausgelöst wird, die gegen die toxischen Gluten- bzw. Gliadin-Peptidsequenzen gerichtet sind. Diese im ersten Blick einfache und durchaus nachvollziehbare Maßnahme, nämlich die Präsentation der toxischen Gluten- bzw. Gliadin-Peptidsequenzen in den späten Endosomen an den MHC-Klasse II Komplexen ist dabei als überaus erfinderisch und neu zu werten und stellt eine Behandlungsmöglichkeit und erstmalige Heilungsmöglichkeit der Zöliakie zur Verfügung, da wie bereits erwähnt, erstmalig die toxischen Peptidsequenzen des Gliadins an den MHC Klasse II Komplexen der späten Endosomen von Enterozyten präsentierbar sind, wodurch eine kontrollierte Immunantwort ausgelöst wird.

Erstmalig ist es nämlich mit dem erfindungsgemäßen Impfstoff möglich, eine Immunantwort im Sinne einer oralen Toleranz auf toxische Peptidsequenzen des Glutens bzw. des Gliadins zu provozieren, die zur Bildung von Antikörpern oder supprimierenden T-Lymphozyten gegen die als ursächlich für die Zöliakie zu beurteilenden toxischen Peptide führt.

In Abkehr zu den derzeitigen Forschungen und Entwicklungen von Präparaten zur Behandlung von Zöliakie, die sich auf das pathogene sekundäre Erscheinungsbild der Zöliakie stützen, nämlich der toxischen Reaktionen und dem Nachweis der toxischen Peptidsequenzen des Glutens bzw. Gliadins in der Lamina propria des Darmepithels von an Zöliakie erkrankten Patienten, gelingt es erstmalig in erfindungsgemäßer Weise, die toxischen Peptidsequenzen in einem Designerpeptid, das aus wenigstens einem Teil eines bakteriellen Toxins konjugiert mit einer toxischen Peptidsequenz des Gliadins besteht, in die späten Endosomen der Enterozyten des Darmepithels zu leiten, um eine kontrollierte Immunantwort auf die toxischen Peptidsequenzen auszulösen, die zur Bildung von Antikörpern oder supprimierenden T-Lymphozyten führt.

Erfindungsgemäß wird das B-Pentamer des Choleratoxins verwendet, das selber zu keiner toxischen Reaktion des Immunsystems führt, aber als Träger der toxischen bzw. immundominanten Peptidsequenz dient, um diese in die späten Endosomen der Enterozyten des Darmepithels zu leiten. Besonders bevorzugt ist dabei das B-Pentamer des Chloleratoxins ohne die toxische A-Fraktion, welches als Schleimhaut-Adjuvans verwendet wird; um als Träger für die toxischen bzw. immundominanten Peptidsequenzen zu dienen.

Als toxische bzw. immundominante Peptidsequenz wird erfindungsgemäß die Peptidsequenz "Pro Gly Glu Glu Glu Pro Phe Pro Pro Glu Glu Pro Tyr Pro Glu Pro Glu Pro Phe Pro" des α-Gliadins (AS 31-49) mit einem Teil des bakteriellen Toxins verknüpft, welche als toxische Peptidsequenz, eine Zottenatrophie verursachend, bekannt

Durch Konjugation der Peptidsequenz des Gliadins mit wenigstens einem Teil des bakteriellen Toxins wird dieses als Antigen präsentiert und führt zu einer Immunantwort durch Bildung von Antikörpern gegen Prolamine, also gegen Zöliakie, und stellt einen Impfstoff gegen Zöliakie dar.

Um Wiederholungen zu vermeiden wird bezüglich der Vorteil le des mit dem nebengeordneten Anspruch 4 beanspruchten Verfahrens, der Verwendung nach Anspruch 6 und des mit Anspruch 7 beanspruchten Designerpeptids auf die Ausführungen zu dem nach Anspruch 1 beanspruchten Impfstoff verwiesen.

Ausführungsbeispiele werden anhand des folgenden Beispiels näher erläutert:

### Beispiel

Es wurden Biopsien aus dem Jejunum von Patienten (1 bis 12 Jahre alt) mit unbehandelter Zöliakie und von erwachsnen Patienten mit einer mindestens sechsmonatigen Glutenfreien Diät genommen. Die Biopsien der Patienten mit unbehandelter Zöliakie dienen als Kontrollen. Die Biopsien der Patienten werden mit Frazers Fraktion (FF) *in vivo* bei 37°C inkubiert oder *in vitro* für 10, 20 und 30 min inkubiert und anschließend mit 5% Paraformaldehyd in 250 mM HEPES-Puffer bei pH 7,4 fixiert.

### Immunfluoreszenz / konfokale Lasermikroskopie

Die Darmbiopsien (gefrorene Schnitte (0,5 µm)) werden mittels eines Ultrakryomikrotoms bei -60°C bis -70°C präpariert. Diese werden mit einem Glas, welches mit poly-L-Lysin überzogen ist, abgedeckt. Die Schnitte werden mit 0,05% Saponin permeabilisiert, um die Signale der intrazellularen Bindungsstellen zu erhöhen, für 1 Stunde mit einem primären Antikörper inkubiert und dann für eine weitere Stunde mit einem zweiten Antikörper bei Raumtemperatur inkubiert. Die Proben können beispielsweise mit einem Zeiss Axioskop Fluoreszenz Mikroskop fotografiert werden.

Zur Darstellung der Kompartimentierung werden HT29-Zellen mit den entsprechenden Gliadin-Peptiden konjugierter oder unkonjugierter Form inkubiert und mit einem polyklonalen oder monoklonalen Gliadin-Antikörper markiert, der an einem zweiten Antikörper gebunden ist, der mit Alexa Fluor 568 verknüpft ist. Die Markerproteine des endosomalen Kompartiments (Cathepsin D, LAMP-2) werden mit einer Alexa Fluor 488 Färbung markiert. Konfokale Bilder können unter Verwendung eines Leica TCS SPII-Mikroskops (Leica Microsystems, Wetzlar, Deutschland) gemacht werden. Die quantitative Auswertung der internalisierten Peptide 31-49 und 31-49-CTB kann mit dem Programm "ImageJ" ausgeführt werden.

### Konjugation des Peptids mit der Aminosäure 31-49 des α-Gliadins mit dem B-Pentamer des Choleratoxins

Das Peptid AS 31-49 des α-Gliadins wird unter Verwendung des heterobifunktionalen "Cross linker"-Reagenz m-Maleinmidobenzoyl-N-hydrosuccimid (MBS - Pierce) mit dem B-Pentamer des Choleratoxins verknüpft. Dazu werden zuerst die Aminogruppen des Trägerproteins durch Inkubation bei Raumtemperatur für 30 min in einem 25-fachen molaren MBS buffer (25 mM MES, pH 6.5, and 150 mM NaCl) inkubiert. Der überschüssige MBS-Puffer wird über eine Säule entfernt. In einem zweiten Schritt werden die Peptide (AS 31-49) in einem molaren Verhältnis von 20:1 zu dem Peptid-Träger-Protein hinzugegeben. Die Verknüpfung an den Cysteinresten der Peptide erfolgt innerhalb von 3 Stunden bei Raumtemperatur. Ungebundene Peptide werden mittels Dialyse gegen PBS entfernt. Die erfolgreiche Konjugation kann mittels Dot Blot-Analyseverfahren erfolgen. Die auf eine Nitrocellulosemembran geblotteten Konjugate können mit den Antikörpern (WBB und R5) gegen Gliadin detektiert werden.

Das so hergestellte Konjugat ist nun bereit, um mit den für Impfstoffe bekannten Trägerstoffen als Impfstoff eingesetzt zu werden.

Die Erfindung wird nachfolgend mit Bezug auf die Zeichnungen noch detaillierter erläutert. Es werden dabei Ausführungsbeispiele gezeigt, die den Schutzbereich der vorgelegten Ansprüche in keiner Weise einschränken sollen.

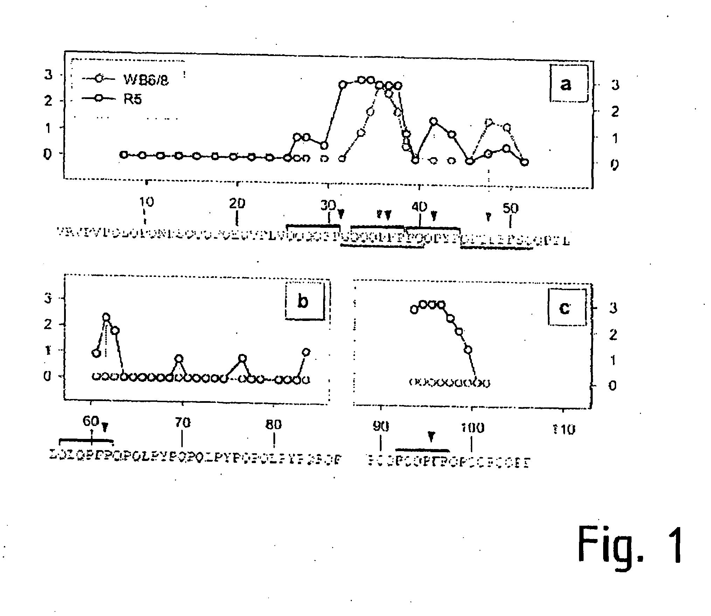

Die Figur 1 zeigt die Reaktivität der monoklonalen Antikörper WB8 und R5 mit Teilsequenzen von Gliadinpeptiden, wobei (a) die Peptidsequenz AS 3-55 des α-Gliadins ist, die mit den Aminosäuren 31 bis 49 als toxische Peptidseqeunz des α-Gliadins charakterisiert ist und die einzige Sequenz darstellt, an die WB8 bindet. Alle anderen Sequenzen, die des α₂-Gliadins (As 56-88) und die des γ-Gliadins (As 89-106) sind lediglich mit dem Antikörper R5 detektierbar. Insofern eignet sich insbesondere der Antikörper WB8 zum Nachweis der toxischen Peptidsequenz AS 31-49 des α-Gliadins.

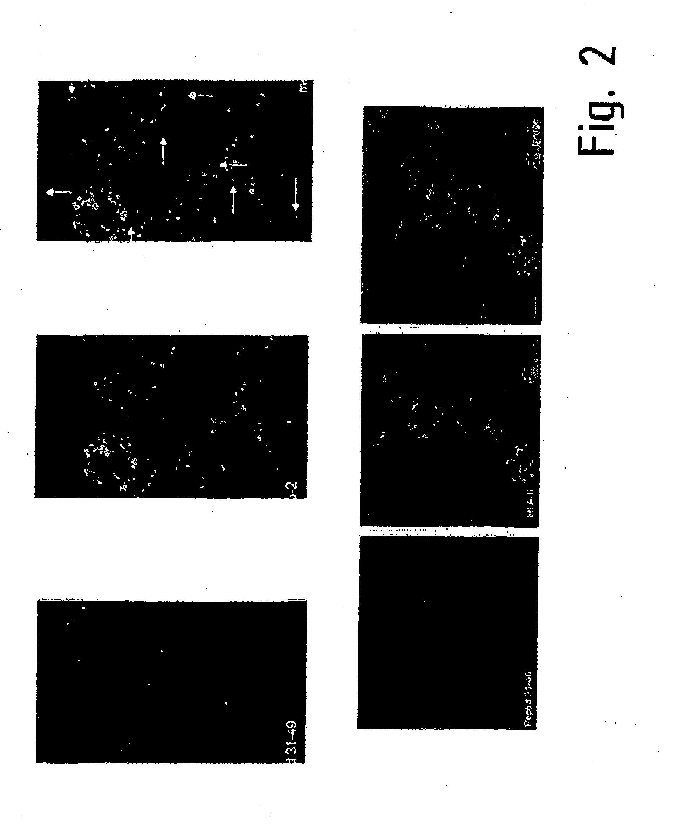

Die Figur 2 zeigt die endosomale Verteilung der synthetischen Peptide in HT29-Zellen, wobei zu erkennen ist, dass die toxischen Peptide nicht in die späten Endosomen gelangen. Im Einzelnen zeigt (A) eine Co-Lokalisation der toxischen Peptide (AS 31-49 des α-Gliadins) mit LAMP-2, (B) die Verteilung der HLA Klasse II Proteine. Entsprechend kann gezeigt werden, dass die toxischen Peptide weder die späten Endosomen noch die HLA Klasse II beinhaltenden Vesikel erreichen. Somit kommt es nicht zur Antigenpräsentierung in den späten Endosomen der Enterozyten und zur Toleranzausprägung gegenüber Gliadin.

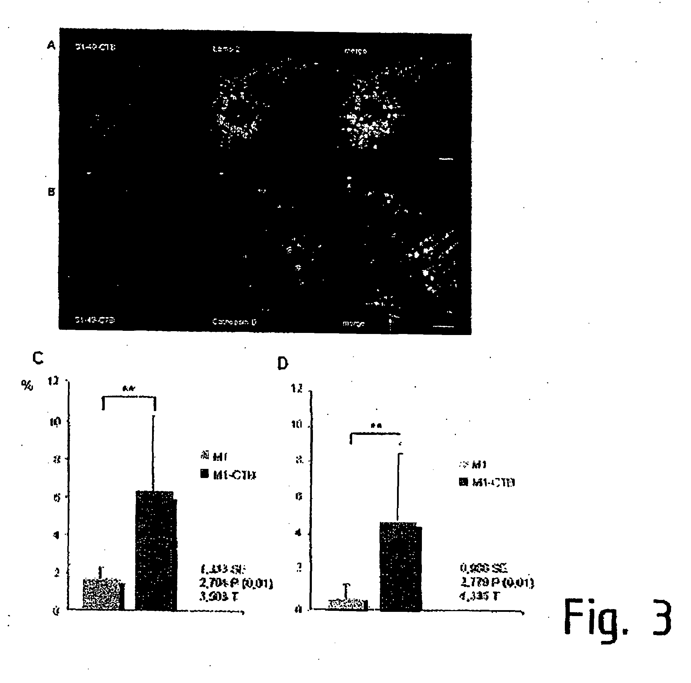

Ein entsprechendes Targeting kann aber mit dem neuen Designerpeptid (CTB-Peptidsequenz-AS 31-49 des α-Gliadins), welches aus der Peptidsequenz AS 31-49 des α-Gliadins konjugiert mit dem B-Pentamer des Choleratoxins besteht, wie in Figur 3 dargestellt, erreicht werden. Dafür wurden HT29 Zellen für 2,5 Stunden mit dem chemisch verknüpften Konjugat CTB-Peptidsequenz-AS 31-49 des α-Gliadins bei 37°C konjugiert. (A) zeigt eine indirekte Immunfluoreszenz mit dem Färbemittel Alexa Fluor 568 (CTB-Peptidsequenz-AS 31-49 des α-Gliadins) und 488 (LAMP-2 und (B) mit Cathepsin D, die beide Markerproteine für späte Endosomen sind, also die Endosomen der Enterozyten markieren). Wie zu erkennen ist, ist das Designerprotein in diesen Kompartimenten colokalisiert. Das bedeutet, dass das B-Pentamer des Choleratoxins als effektives Trägerprotein die toxische Peptidsequenz des α-Gliadins (As 31-49) in die späten endosomen geschleust hat und somit wirkungsvoll für eine Immunantwort den MHC Klasse II-Proeteinen präsentiert wird.

Das belegt, dass das Designerpeptid, also das Konjugat, bestehend aus dem B-Pentamer des Choleratoxins und der toxischen Peptidsequenz des α-Gliadins (As 31-49) als wirksamer Bestandteil eines Impfstoffes gegen Zöliakie verwendbar ist. Wie wirkungsvoll dabei das B-Pentamer des Choleratoxins die toxische Peptidsequenz des α-Gliadins (As 31-49) in die späten Endosomen leitet, zeigen (C) und (D) der Figur 3, denn die Konjugation der toxischen Peptidsequenz des α-Gliadins (As 31-49) mit dem B-Pentamer des Choleratoxins führt zu einer stark erhöhten Internalisation des toxischen peptides in den späten Endosomen gegenüber nicht konjugierten toxischen Peptiden (C). Zudem ist die Effizienz, also die Wahrscheinlichkeit, dass die toxischen Peptide in die späten Endosomen gelangen und dort zu einer kontrollierten Immunantwort führen zusammen mit dem B-Pentamer des Choleratoxins als Konjugat extrem erhöht.

Das anhand der Figuren beschriebene Ausführungsbeispiel belegt, dass es möglich ist, toxische Peptidsequenzen des Gliadins, die normalerweise nicht in die späten Endosomen von Enterozyten gelangen und somit auch keine kontrollierte Immunantwort unter Bildung von Antikörpern oder supprimierenden T-Lymphozyten auslösen, als Konjugat mit einem Teil eines bakteriellen Toxins, dass als Trägerprotein (Adjuvans) fungiert, in die späten Endosomen der Enterozyten zu leiten, um als Antigen den MHC Klasse II Komplexen präsentiert zu werden, also von Enterozyten präsentierbar sind. Damit ist belegt, dass die vorliegende Erfindung technisch ausführbar ist, dass die Möglichkeit besteht, einen Impfstoff unter Verwendung eines Designerpeptids, bestehend aus wenigstens einem Teil eines bakteriellen Toxins und mindestens einem daran konjugierten toxischen Peptids des Glutens bzw. Gliadins gegen Zöliakie herzustellen und diesen zur Behandlung und Heilung von Zöliakie zu verwenden.

## Patentansprüche

1. Impfstoff zur Anwendung in der Behandlung von Zöliakie, welcher als wirksamen Bestandteil mindestens eine mit einem Teil eines bakteriellen Toxins konjugierte unverzweigte toxische Peptidsequenz eines Gliadins enthält, wobei die
unverzweigte toxische Peptidsequenz Pro Gly Glu Glu Glu Pro Phe Pro Pro Glu Glu Pro Tyr Pro Glu Pro Glu Pro Phe Pro des α-Gliadins ist und das bakterielle Toxin das B-Pentamer des Choleratoxins ist.

2. Impfstoff zur Behandlung von Zöliakie nach Anspruch 1, **dadurch gekennzeichnet, dass** das bakterielle Toxin als Adjuvans zur Antigenpräsentierung der toxischen Peptidsequenz in den späten Endosomen der Enterozyten des Darmepithels dient.

3. Impfstoff zur Behandlung von Zöliakie nach einem der Ansprüche 1 bis 2, **dadurch gekennzeichnet, dass** die Behandlung eine aktive Impfung ist.

4. Verfahren zur Herstellung eines Impfstoffes zur Behandlung von Zöliakie nach den Ansprüchen 1 bis 3, **dadurch gekennzeichnet, dass** die unverzweigte toxische Peptidsequenz Pro Gly Glu Glu Pro Phe Pro Pro Glu Glu Pro Tyr Pro Glu Pro Glu Pro Phe Pro mit wenigstens einem Teil eines bakteriellen Toxin verknüpft wird, wobei das bakterielle Toxin das B-Pentamer des Chloleratoxins ist.

5. Verfahren zur Herstellung eines Impfstoffes zur Behandlung von Zöliakie nach Anspruch 4, **dadurch gekennzeichnet, dass** eine Untereinheit des bakteriellen Toxins, die einen tolerogenen Effekt von Antigenen aufweist, ohne die toxische Fraktion des bakteriellen Toxins mit der Peptidsequenz konjugiert wird.

6. Verwendung mindestens der mit dem B-Pentamer des Chloleraxins konjugierten unverzweigten toxischen Peptidsequenz Pro Gly Glu Glu Glu Pro Phe Pro Pro Glu Glu Pro Tyr Pro Glu Pro Glu Pro Phe Pro zur Herstellung eines Impfstoffes zur spezifischen Aktivierung des Immunsystems gegen Prolamine.

7. Designerpeptid, bestehend aus der Aminosäuresequenz Pro Gly Glu Glu Glu Pro Phe Pro Pro Glu Glu Pro Tyr Pro Glu Pro Glu Pro Phe Pro des α-Gliadins, die mit dem B-Pentamer des Choleratoxins konjugiert ist.

8. Designerpeptid nach Anspruch 7 zur Herstellung eines Therapeutikums oder Impfstoffes, das zur Behandlung von Zöliakie einsetzbar ist.

## Claims

1. Vaccine for use in the treatment of coeliac disease, which vaccine contains, as active component, at least one unbranched toxic peptide sequence of a gliadin that is conjugated to a part of a bacterial toxin, wherein the unbranched toxic peptide sequence is Pro Gly Glu Glu Glu Pro Phe Pro Pro Glu Glu Pro Tyr Pro Glu Pro Glu Pro Phe Pro
of the α-gliadin and the bacterial toxin is the B-pentamer of the cholera toxin.

2. Vaccine for treating coeliac disease according to Claim 1, **characterized in that** the bacterial toxin serves as adjuvant for antigen presentation of the toxic peptide sequence in the late endosomes of the enterocytes of the intestinal epithelium.

3. Vaccine for treating coeliac disease according to either of Claims 1 to 2, **characterized in that** the treatment is an active inoculation.

4. Method for producing a vaccine for treating coeliac disease according to Claims 1 to 3, **characterized in that** the unbranched toxic peptide sequence Pro Gly Glu Glu Glu Pro Phe Pro Pro Glu Glu Pro Tyr Pro Glu Pro Glu Pro Phe Pro is linked to at least one part of a bacterial toxin, wherein the bacterial toxin is the B-pentamer of the cholera toxin.

5. Method for producing a vaccine for treating coeliac disease according to Claim 4, **characterized in that** a subunit of the bacterial toxin that has a tolerogenic effect of antigens, without the toxic fraction of the bacterial toxin, is conjugated to the peptide sequence.

6. Use of at least the unbranched toxic peptide sequence Pro Gly Glu Glu Glu Pro Phe Pro Pro Glu Glu Pro Tyr Pro Glu Pro Glu Pro Phe Pro conjugated to the B-pentamer of the cholera toxin for producing a vaccine for the specific activation of the immune system against prolamines.

7. Designer peptide consisting of the amino acid sequence Pro Gly Glu Glu Glu Pro Phe Pro Pro Glu Glu Pro Tyr Pro Glu Pro Glu Pro Phe Pro
of the α-gliadin which is conjugated to the B-pentamer of the cholera toxin.

8. Designer peptide according to Claim 7 for producing a therapeutic agent or vaccine which can be used for treating coeliac disease.

## Revendications

1. Vaccin pour une application dans le traitement de la maladie coeliaque, lequel contient comme composant efficace au moins une séquence peptidique toxique non ramifiée d'une gliadine conjuguée avec une partie d'une toxine bactérienne, sachant que la séquence peptidique toxique non ramifiée est Pro Gly Glu Glu Glu Pro Phe Pro Pro Glu Glu Pro Tyr Pro Glu Pro Glu Pro Phe Pro de la α-gliadine et la toxine bactérienne est le β-pentamère de la toxine du choléra.

2. Vaccin pour le traitement de la maladie coeliaque selon la revendication 1, **caractérisé en ce que** la toxine bactérienne sert d'adjuvant pour la présentation d'antigènes de la séquence peptidique toxique dans les endosomes tardifs des entérocytes de l'épithélium intestinal.

3. Vaccin pour le traitement de la maladie coeliaque selon l'une quelconque des revendications 1 à 2, **caractérisé en ce que** le traitement est une vaccination active.

4. Procédé de préparation d'un vaccin pour le traitement de la maladie coeliaque selon l'une quelconque des revendications 1 à 3, **caractérisé en ce que** la séquence peptidique toxique non ramifiée Pro Gly Glu Glu Glu Pro Phe Pro Pro Glu Glu Pro Tyr Pro Glu Pro Glu Pro Phe Pro est enchaînée à au moins une partie d'une toxine bactérienne, la toxine bactérienne étant le β-pentamère de la toxine du choléra.

5. Procédé de préparation d'un vaccin pour le traitement de la maladie coeliaque selon la revendication 4, **caractérisé en ce qu'**une sous-unité de la toxine bactérienne qui présente un effet tolérogénique d'antigène est conjuguée sans la fraction toxique de la toxine bactérienne avec la séquence peptidique.

6. Utilisation d'au moins la séquence peptidique toxique non ramifiée Pro Gly Glu Glu Glu Pro Phe Pro Pro Glu Glu Pro Tyr Pro Glu Pro Glu Pro Phe Pro conjuguée avec le β-pentamère de la toxine du choléra à la préparation d'un vaccin pour l'activation spécifique du système immunitaire contre les prolamines.

7. Peptide artificiel, consistant en la séquence d'acides aminés Pro Gly Glu Glu Glu Pro Phe Pro Pro Glu Glu Pro Tyr Pro Glu Pro Glu Pro Phe Pro de la α-gliadine qui est conjugué avec le β-pentamère de la toxine du choléra.

8. Peptide artificiel selon la revendication 7 pour la préparation d'un médicament thérapeutique ou d'un vaccin qui est utilisable pour le traitement de la maladie coeliaque.
